# FASCICULE DE BREVET EUROPEEN

(11) **EP 2 920 160 B1**
(45) Date de publication et mention de la délivrance du brevet: **27.07.2022**
(21) Numéro de dépôt: 13815028.9
(22) Date de dépôt: 14.11.2013
(51) Int. Cl.: C07D 307/80, C07C 37/72, C07C 39/21, C07H 15/203

(54) **PROCEDE D'OBTENTION DE POLYPHENOLS A PARTIR D'UNE MATIERE PREMIERE VEGETALE EN CONTENANT**
VERFAHREN ZUR GEWINNUNG VON POLYPHENOLEN AUS PFLANZENMATERIAL DAMIT
METHOD FOR OBTAINING POLYPHENOLS FROM A VEGETABLE RAW MATERIAL CONTAINING SAME

(30) Priorité: 16.11.2012 FR 1260916
(43) Date de publication de la demande: 23.09.2015
(73) Titulaire: EXINNOV, 33000 Bordeaux (FR)
(72) Inventeur: SOUM, Stéphane, F-86440 Migne-Auxances (FR); PICCIRILLI, Antoine, F-86000 Poitiers (FR); BATAILLE, Frédéric, F-86800 Sevres-Anxaumont (FR)
(74) Mandataire: Aquinov
(86) Numéro de dépôt international: PCT/FR2013/052739
(87) Numéro de publication internationale: WO 2014/076425

(56) Documents cités:
- US-A1- 2009 191 294
- HA, DO THI ET AL: "Stilbenes and oligostilbenes from leaf and stem of Vitis amurensis and their cytotoxic activity", ARCHIVES OF PHARMACAL RESEARCH , 32(2), 177-183 CODEN: APHRDQ; ISSN: 0253-6269, 2009, XP002711995,
- PERALBO-MOLINA, ANGELA ET AL: "Comparison of extraction methods for exploitation of grape skin residues from ethanol distillation", TALANTA , 101, 292-298 CODEN: TLNTA2; ISSN: 0039-9140, 3 octobre 2012 (2012-10-03), XP002711996,

## Description

La présente invention concerne l'obtention de polyphénols à partir d'une matière première végétale en contenant.

Les polyphénols sont des molécules naturelles présentant un intérêt croissant dans de nombreux domaines. Leur propriétés antioxydantes permettent en particulier des applications thérapeutiques notamment pour la prévention et le traitement de cancers (comme illustré par exemple dans YUN & al. « Pro-and Anti-angiogenesis effects of Resveratrol » In vivo 21 pages 365-370), de maladies inflammatoires, cardiovasculaires et neurodégénératives. Elles sont également utilisées dans l'industrie cosmétique comme cela est décrit par exemple dans la demande EP1820492, ou en agriculture comme stimulateur de défenses naturelles, hormone de croissance ou biopesticide.

Il existe plusieurs sources naturelles végétales de polyphénols, comme les produits de la vigne, le pin, le thé, le cacao, la pomme, le soja, le lin, les fruits rouges tels que la myrtille, la cranberry, les agrumes, l'hammamelis, les noix de galle, le sumac, les bruyères, les écorces de quebracho, de châtaigner et de chêne, etc. Les polyphénols, comme les oligomères procyanidoliques, stilbènes, lignanes, catéchines, chalcones, flavonoïdes, isoflavonoïdes, acides phénoliques, le résveratrol, la viniférine, le piceid par exemple, leur permette de se défendre contre des agents pathogènes.

Actuellement, les procédés d'extraction de polyphénols, reposent sur le principe de la macération dans un solvant adapté. Ces procédés, notamment décrits dans les demandes FR2795965, EP1787970, WO2007/017037, US2009/191294 ou encore la publication HA, DO THI ET AL « Stilbenes and oligostilbenes from leaf and stem of Vitis amurensis and their cytotoxic activity » nécessitent une mise en contact à température ambiante ou à chaud, l'utilisation d'une quantité importante de solvant et une étape de purification dans une solution alcoolique est nécessaire après extraction. Ils sont réalisés en discontinu, sont longs, couteux et demande une énergie importante pour des rendements d'extraction faibles.

On connaît également la demande FR2967586 qui décrit un procédé d'extraction de polyphénols à partir de marc de raisin, comprenant une étape de traitement électrique par puissance pulsée, et une diffusion des polyphénols dans un solvant. Il s'agit également d'un procédé discontinu, mis en œuvre sur des unités de très faible capacité et représentant des investissements élevés.

La publication PERALBO-MOLINA, ANGELA ET AL « Comparison of extraction methods for exploitation of grape skin residues from ethanol distillation» décrit aussi des procédés d'extraction à partir de résidus de peau de raisin provenant de la distillation éthanolique. Quatre méthodes sont comparées mais aucune n'est satisfaisante.

Enfin, la demande WO02064536 décrit un procédé d'extraction de polyphénols par flash détente et chromatographie préparative. Ce procédé de flash détente est plutôt adapté à des matières premières fragiles et de faible tenue mécanique, et non à des matières fibreuses solides telles que les sarments, les écorces, les pépins, les tourteaux oléagineux.

La présente invention a pour objectif de pallier les inconvénients des procédés existants en proposant un procédé d'obtention de polyphénols à partir d'une matière première végétale en contenant, efficace, nécessitant l'utilisation d'une faible quantité de solvant, moins couteux et pouvant être réalisé en continu.

A cet effet l'invention a pour objet un procédé d'obtention de polyphénols à partir d'une matière première en contenant, comprenant au moins les étapes suivantes :
- éventuellement séchage de la matière première, l'étape de séchage étant réalisée de façon à ce que la teneur en humidité soit inférieure ou égale à 30%,
- broyage de la matière première séchée ou sèche naturellement,
- défibrage de la matière première broyée dans une extrudeuse en présence d'un solvant,
- séparation de la phase soluble et des fibres, réalisée à l'aide d'un fourreau pressoir monté au niveau d'une zone de compression de l'extrudeuse ou réalisée en sortie d'extrudeuse par filtration sous azote ou pressage,
- récupération de la phase soluble, extrait brut, contenant les polyphénols.

Avantageusement le procédé selon l'invention permet d'obtenir des polyphénols en quantité importante avec une faible quantité de solvant. Pour certains polyphénols, par exemple pour la viniférine obtenue à partir de co-produits de la vigne, les quantités obtenues sont bien plus importantes qu'avec les procédés antérieurs.

L'invention est maintenant décrite en détail.

L'objet de l'invention est un procédé d'obtention de polyphénols à partir d'une matière première en contenant.

Cette matière première peut être notamment choisie parmi les écorces de pin, les feuilles de thé, les fèves de cacao et les produits et/ou coproduits de la vigne. Par produits et/ou coproduits de la vigne, on entend les raisins, notamment pépins et peaux, les sarments de vigne, les feuilles de vigne, les ceps de vignes, les rafles, le marc de raisin et les fibres issues de la production de jus de raisin. Ces différentes matières premières sont désignées comme des produits et/ou coproduits dans la vigne dans la présente description.

Le procédé selon l'invention comprend au moins les étapes suivantes :
- éventuellement séchage de la matière première contenant des polyphénols, l'étape de séchage étant réalisée de façon à ce que la teneur en humidité soit inférieure ou égale à 30%,
- broyage de la matière première séchée ou sèche naturellement,
- défibrage de la matière première broyée dans une extrudeuse en présence d'un solvant,
- séparation de la phase soluble et des fibres, réalisée à l'aide d'un fourreau pressoir monté au niveau d'une zone de compression de l'extrudeuse ou réalisée en sortie d'extrudeuse par filtration sous azote ou pressage.
- récupération de la phase soluble, extrait brut, contenant les polyphénols.

Le séchage est nécessaire si la matière première est fraiche, c'est-à-dire si elle contient plus de 30% d'humidité. Si la matière première a séché naturellement et est mature, l'étape de séchage n'est pas nécessaire.

Il est important que la matière première soit séchée ou sèche pour assurer un débit important lors du broyage et éviter le bourrage de la matière dans l'extrudeuse.

Le séchage peut être réalisé en étuve.

La teneur en humidité dans la matière première après séchage ou sèche naturellement, doit être inférieure ou égale à 30%.

La matière première séchée ou sèche est ensuite broyée.

Le broyage peut être effectué par tout moyen connu, par exemple à l'aide d'un broyeur à couteaux ou à marteaux, ou encore par cryobroyage.

Le broyage doit être effectué de façon à obtenir une granulométrie de la matière première adaptée à la taille de l'extrudeuse : si les particules sont trop grosses il y a un risque de bourrage de l'extrudeuse et si les particules sont trop fines, l'effet mécanique du défibrage ne sera pas optimal. Par exemple, pour une extrudeuse bivis corotative dont le rapport longueur/diamètre est compris entre 1 et 100 et plus particulièrement entre 20 et 40 et dont l'entraxe est de 21 mm, la granulométrie des particules doit être comprise entre 1 et 10 mm, plus particulièrement entre 1 et 5 mm.

Après broyage, la matière première est défibrée dans une extrudeuse en présence d'un solvant.

Préférentiellement, la matière première broyée est mélangée à un solvant d'extraction et l'ensemble est introduit dans une extrudeuse, par exemple à l'aide d'un doseur gravimétrique.

L'extrudeuse est préférentiellement une extrudeuse bivis corotative.

Une extrudeuse est composée principalement d'une ou plusieurs vis sans fin tournant à vitesse déterminée à l'intérieur d'un fourreau cylindrique régulé en température.

L'extrudeuse bivis corotative est constituée de deux vis copénétrantes tournant dans le même sens et d'un fourreau enveloppant ces deux vis. Celles-ci sont identiques et sont composées de modules qui comprennent un élément de convoyage du matériau muni de fenêtres ou de fentes taillées dans les filets, cet élément étant nommé contrefilet. L'extrudeuse bivis permet de réaliser en une seule étape une succession d'opérations : défibrage, lavage, réaction chimique, extrusion.

Dans le procédé selon l'invention, le défibrage est donc réalisé par la conjugaison des actions de compression et de cisaillement dans l'extrudeuse.

Cela permet de séparer les fibres et le solvant d'extraction peut extraire plus facilement et plus rapidement des polyphénols et la quantité de solvant à utiliser est par conséquent plus faible que dans les procédés antérieurs.

De façon préférée, le solvant utilisé pour l'étape de défibrage est choisi parmi l'eau, l'acétate d'éthyle, les alcools (méthanol, éthanol, isopropanol, butanol, etc.), les cétones (acétone, diéthylcétone, etc.), les diols (propane-1,2-diol, propane-1,3-diol, etc.), les glycols (dipropylèneglycol, etc.). Ces solvants sont utilisés purs ou en mélanges.

Le rapport solvant / matière première séchée ou sèche est préférentiellement compris entre 100 et 1 000%, préférentiellement entre 200 et 500%.

L'étape d'extrusion peut être réalisée à une température comprise entre la température ambiante et 180°C. Le temps de mélange préalable à l'introduction dans l'extrudeuse peut durer de 1 minute à 4 heures, et le temps de latence de 0 à 2 heures.

En fonction du solvant d'extraction utilisé, il est possible d'ajuster le pH en ajoutant une solution basique ou acide audit solvant, pour améliorer encore l'extraction.

Après cette étape d'extraction dynamique par extrusion, il est nécessaire de séparer la partie soluble des fibres.

La séparation de la partie soluble et des fibres peut s'effectuer à l'aide d'un fourreau pressoir, monté au niveau d'une zone de compression dans l'extrudeuse.

Il est également possible de les séparer en sortie d'extrudeuse par des techniques connues de filtration sous azote ou de pressage.

La phase soluble est ensuite récupérée. Il s'agit de l'extrait brut contenant les polyphénols.

Après cette étape, le procédé selon l'invention comprend préférentiellement une étape de concentration en polyphénols de l'extrait brut obtenu dans la phase soluble. Cette concentration en polyphénols peut être réalisée par évaporation du solvant en chauffant ou par lyophilisation.

L'extrait brut récupéré contient un mélange de polyphénols. Il peut être utilisé tel quel en cocktail ou les différents polyphénols peuvent être séparés et purifiés en particulier par chromatographie préparative sur colonne ou par chromatographie de partage centrifuge (CPC).

Le procédé selon l'invention peut donc comprendre une étape supplémentaire de séparation des polyphénols de l'extrait. Par exemple, lorsque la matière première est un produit ou coproduit de la vigne, on cherchera à récupérer la viniférine, le transresvératrol et la piceid.

Selon un mode de réalisation, après extraction des polyphénols, la fraction solide qui contient encore la cellulose qui n'est pas dégradée, peut être utilisée pour la production de jus sucrés par hydrolyse de la cellulose en glucose, et ensuite la production d'éthanol par fermentation.

Avantageusement, le procédé selon l'invention est simple, continu, économique et nécessite l'utilisation de peu de solvant en comparaison aux procédés antérieurs. En outre, il permet de récupérer des quantités importantes de polyphénols. Pour la viniférine en particulier, les rendements obtenus sont largement supérieurs à ceux obtenus jusqu'alors.

L'invention est à présent illustrée par des essais réalisés avec le procédé selon l'invention et des essais comparatifs avec des méthodes antérieures.

### 1. Exemple de procédé selon l'invention pour l'extraction de viniférine et de trans-résveratrol à partir de sarments de vigne de Melon de Bourgogne

Des sarments de vigne de Melon de Bourgogne séchés sont broyés à 1, 4 ou 8mm par un broyeur à couteaux.

Les sarments broyés sont mélangés pendant 15 minutes à un solvant d'extraction constitué de 80% d'éthanol et de 20% d'eau.

Le mélange est ensuite introduit à l'aide d'un doseur gravimétrique dans une extrudeuse bivis corotative Clextral BC21 à 60°C. La vitesse de rotation des vis est de 100 tours minute et le débit du doseur de 2 kg/h.

La séparation du solvant et des fibres est réalisée à l'aide d'un fourreau pressoir monté au niveau d'une zone de compression.

On détermine ensuite la quantité de viniférine et de transresvératrol présents dans l'extrait brut récupéré, par chromatographie HPLC.

Les résultats obtenus sont présentés dans le tableau 1.

**Tableau 1**

| Temps de latence après la taille des vignes | 16 semaines | | | 24 heures |
|---|---|---|---|---|
| Granulométrie, mm | 1 | 4 | 8 | 8 |
| Solvant d'extraction (V/V) | Ethanol / Eau (80/20) | | | |
| Température d'extraction, °C Atmosphère d'extraction | 60 Azote | | | |
| Evaporation | Evaporateur rotatif sous azote | | | |
| Teneur en trans-resveratrol, ppm | 764 | 915 | 387 | 207 |
| Teneur en viniférine, ppm | 5482 | 8170 | 6440 | 2035 |

### 2. Résultats comparatifs pour l'extraction de viniférine et transrésvératrol avec le procédé selon l'invention et un procédé de l'art antérieur à partir de sarments de vigne de Melon de Bourgogne

Deux essais comparatifs sont réalisés :
- un procédé de l'art antérieur : extrusion dynamique par infusion,
- le procédé selon l'invention.

### Extrusion dynamique par infusion :

Les sarments sont broyés à 1mm et sont introduits dans un réacteur tricol muni d'un système d'agitation et d'un système de bullage sous azote. L'extraction à reflux est réalisée pendant 2 heures avec un solvant constitué de 80% d'éthanol et de 20% d'eau, à 60°C.

La séparation du solvant et des fibres est réalisée sur un filtre presse.

### Procédé selon l'invention :

Des sarments de vigne de Melon de Bourgogne séchés sont broyés à 4mm par un broyeur à couteaux.

Les sarments broyés sont mélangés pendant 15 minutes à un solvant d'extraction constitué de 80% d'éthanol et de 20% d'eau.

Le mélange est ensuite introduit à l'aide d'un doseur gravimétrique dans une extrudeuse bivis corotative Clextral BC21 à température ambiante. La vitesse de rotation des vis est de 100 tours minute et le débit du doseur de 2 kg/h.

La séparation du solvant et des fibres est réalisée à l'aide d'un fourreau pressoir monté au niveau d'une zone de compression.

Dans les deux cas, on détermine ensuite la quantité de viniférine et de transresvératrol présents dans l'extrait brut récupéré, par chromatographie HPLC.

Les résultats obtenus sont présentés dans le tableau 2.

**Tableau 2**

| Procédé | Procédé selon l'invention | Extraction dynamique par infusion |
|---|---|---|
| Granulométrie, mm | 4 | 1 |
| Température consigne d'extrusion, °C | T ambiante | 60 |
| Solvant d'extraction (V/V) | Ethanol / Eau (80/20) | Ethanol / Eau (80/20) |
| Teneur en transresvératrol, ppm | 984 | 764 |
| Teneur en viniférine, ppm | 7 400 | 5 482 |

## Revendications

1. Procédé d'obtention de polyphénols à partir d'une matière première végétale en contenant, **caractérisé en ce qu'**il comprend au moins les étapes suivantes :
- éventuellement séchage de la matière première contenant des polyphénols, l'étape de séchage étant réalisée de façon à ce que la teneur en humidité soit inférieure ou égale à 30%,
- broyage de la matière première séchée ou sèche naturellement,
- défibrage de la matière première broyée dans une extrudeuse en présence d'un solvant,
- séparation de la phase soluble et des fibres, réalisée à l'aide d'un fourreau pressoir monté au niveau d'une zone de compression de l'extrudeuse ou réalisée en sortie d'extrudeuse par filtration sous azote ou pressage,
- récupération de la phase soluble, extrait brut, contenant les polyphénols.

2. Procédé d'obtention de polyphénols selon la revendication 1, **caractérisé en ce qu'**il comprend également une étape de concentration en polyphénols de l'extrait brut obtenu dans la phase soluble.

3. Procédé d'obtention de polyphénols selon la précédente revendication, **caractérisé en ce que** la concentration en polyphénols de l'extrait brut est réalisée par évaporation du solvant en chauffant ou par lyophilisation.

4. Procédé d'obtention de polyphénols selon l'une des précédentes revendications, **caractérisé en ce que** le rapport solvant / matière première dans l'extrudeuse est compris entre 100 et 1 000% en poids.

5. Procédé d'obtention de polyphénols selon l'une des précédentes revendications, **caractérisé en ce que** le solvant est choisi parmi l'eau, l'acétate d'éthyle, les alcools, les cétones, les diols, les glycols et leurs mélanges.

6. Procédé d'obtention de polyphénols selon l'une des précédentes revendications, **caractérisé en ce que** le solvant et la matière première broyée sont mélangés avant d'être introduits dans l'extrudeuse pendant 1 minute à 4 heures.

7. Procédé d'obtention de polyphénols selon l'une des précédentes revendications, **caractérisé en ce que** la température dans l'extrudeuse est comprise entre la température ambiante et 180°C.

8. Procédé d'obtention de polyphénols selon l'une des précédentes revendications, **caractérisé en ce que** la séparation de la phase soluble des fibres est réalisée dans l'extrudeuse à l'aide d'un fourreau pressoir monter au niveau d'une zone de compression.

9. Procédé d'obtention de polyphénols selon l'une des revendications 1 à 6, **caractérisé en ce que** la séparation de la phase soluble des fibres est réalisée après extrusion, par filtration sous azote ou par pressage.

10. Procédé d'obtention de polyphénols selon l'une des précédentes revendications, **caractérisé en ce que** la matière première est choisie parmi les écorces de pin, les feuilles de thé, les fèves de cacao et les produits et/ou coproduits de la vigne.

11. Procédé d'obtention de polyphénols selon l'une des précédentes revendications, **caractérisé en ce que** la matière première est un produit et/ou coproduit de la vigne et **en ce que** les polyphénols sont des stilbènes.

12. Procédé d'obtention de polyphénols selon la précédente revendication, **caractérisé en ce que** les polyphénols sont choisis parmi la viniférine, le transresvératrol et la piceid.

## Patentansprüche

1. Verfahren zum Gewinnen von Polyphenolen aus einem pflanzlichen Rohstoff, der sie enthält, **dadurch gekennzeichnet, dass** es mindestens die folgenden Schritte umfasst:
- optional Trocknen des Rohstoffs, der Polyphenole enthält, wobei der Trocknungsschritt so durchgeführt wird, dass der Feuchtigkeitsgehalt weniger als oder gleich 30 % beträgt,
- Mahlen des getrockneten oder naturtrockenen Rohstoffs,
- Zerfasern des gemahlenen Rohstoffs in einem Extruder in Gegenwart eines Lösungsmittels,
- Trennen der löslichen Phase und der Fasern, das mithilfe einer Presshülse durchgeführt wird, die an einer Kompressionszone des Extruders angebracht ist, oder das an einem Extruderausgang durch Filtern unter Stickstoff oder Pressen durchgeführt wird,
- Rückgewinnen eines Rohextrakts, der die Polyphenole enthält, aus der löslichen Phase.

2. Verfahren zum Gewinnen von Polyphenolen nach Anspruch 1, **dadurch gekennzeichnet, dass** es ebenfalls einen Konzentrationsschritt von Polyphenolen des Rohextrakts umfasst, die in der löslichen Phase gewonnen werden.

3. Verfahren zum Gewinnen von Polyphenolen nach dem vorhergehenden Anspruch, **dadurch gekennzeichnet, dass** die Konzentration von Polyphenolen des Rohextrakts durch Verdampfen des Lösungsmittels unter Erwärmen oder durch Gefriertrocknen durchgeführt wird.

4. Verfahren zum Gewinnen von Polyphenolen nach einem der vorhergehenden Ansprüche, **dadurch gekennzeichnet, dass** das Verhältnis Lösungsmittel/Rohstoff in dem Extruder zwischen 100 und 1000 Gew.-% liegt.

5. Verfahren zum Gewinnen von Polyphenolen nach einem der vorhergehenden Ansprüche, **dadurch gekennzeichnet, dass** das Lösungsmittel aus Wasser, Ethylacetat, Alkoholen, Ketonen, Diolen, Glykolen und ihren Mischungen ausgewählt ist.

6. Verfahren zum Gewinnen von Polyphenolen nach einem der vorhergehenden Ansprüche, **dadurch gekennzeichnet, dass** das Lösungsmittel und der gemahlene Rohstoff vor dem Einbringen in den Extruder 1 Minute bis 4 Stunden lang gemischt werden.

7. Verfahren zum Gewinnen von Polyphenolen nach einem der vorhergehenden Ansprüche, **dadurch gekennzeichnet, dass** die Temperatur in dem Extruder zwischen der Raumtemperatur und 180 °C liegt.

8. Verfahren zum Gewinnen von Polyphenolen nach einem der vorhergehenden Ansprüche, **dadurch gekennzeichnet, dass** das Trennen der löslichen Phase von den Fasern in dem Extruder mithilfe einer Presshülse durchgeführt wird, die an der Kompressionszone angebracht ist.

9. Verfahren zum Gewinnen von Polyphenolen nach einem der Ansprüche 1 bis 6, **dadurch gekennzeichnet, dass** das Trennen der löslichen Phase von den Fasern nach dem Extrudieren durch Filtern unter Stickstoff oder durch Pressen durchgeführt wird.

10. Verfahren zum Gewinnen von Polyphenolen nach einem der vorhergehenden Ansprüche, **dadurch gekennzeichnet, dass** der Rohstoff aus Pinienrinden, Teeblättern, Kakaobohnen und Weinprodukten und/oder -nebenprodukten ausgewählt ist.

11. Verfahren zum Gewinnen von Polyphenolen nach einem der vorhergehenden Ansprüche, **dadurch gekennzeichnet, dass** der Rohstoff ein Weinprodukt und/oder - nebenprodukt ist und dass die Polyphenole Stilbene sind.

12. Verfahren zum Gewinnen von Polyphenolen nach dem vorhergehenden Anspruch, **dadurch gekennzeichnet, dass** die Polyphenole aus Viniferin, Transresveratrol und Piceid ausgewählt sind.

## Claims

1. Method for obtaining polyphenols from a plant raw material containing said polyphenols, **characterized in that** it comprises at least the following steps:
- optionally drying the raw material containing polyphenols, the drying step being carried out such that the moisture content is less than or equal to 30%,
- grinding the dried or naturally dry raw material,
- removing fibers from the ground raw material in an extruder in the presence of a solvent,
- separating the soluble phase and the fibers using a press sleeve mounted in a compression region of the extruder or separating said phase and fibers at the extruder outlet by filtration under nitrogen or pressing,
- recovering the soluble phase, the crude extract, which contains the polyphenols.

2. Method for obtaining polyphenols according to claim 1, **characterized in that** it also comprises a step of concentrating polyphenols from the crude extract obtained in the soluble phase.

3. Method for obtaining polyphenols according to the preceding claim, **characterized in that** polyphenols are concentrated from the crude extract by evaporating the solvent by heating or by freeze-drying.

4. Method for obtaining polyphenols according to any of the preceding claims, **characterized in that** the solvent/raw material ratio in the extruder is between 100 and 1000% by weight.

5. Method for obtaining polyphenols according to any of the preceding claims, **characterized in that** the solvent is selected from water, ethyl acetate, alcohols, ketones, diols, glycols and mixtures thereof.

6. Method for obtaining polyphenols according to any of the preceding claims, **characterized in that** the solvent and the ground raw material are mixed before being introduced into the extruder for 1 minute to 4 hours.

7. Method for obtaining polyphenols according to any of the preceding claims, **characterized in that** the temperature in the extruder is between room temperature and 180°C.

8. Method for obtaining polyphenols according to any of the preceding claims, **characterized in that** the soluble phase is separated from the fibers in the extruder using a press sleeve mounted in a compression region.

9. Method for obtaining polyphenols according to any of claims 1 to 6, **characterized in that** the soluble phase is separated from the fibers after extrusion by filtration under nitrogen or by pressing.

10. Method for obtaining polyphenols according to any of the preceding claims, **characterized in that** the raw material is selected from pine bark, tea leaves, cocoa beans and vine products and/or co-products.

11. Method for obtaining polyphenols according to any of the preceding claims, **characterized in that** the raw material is a vine product and/or co-product and **in that** the polyphenols are stilbenes.

12. Method for obtaining polyphenols according to the preceding claim, **characterized in that** the polyphenols are selected from viniferine, trans-resveratrol and piceid.
